# EUROPEAN PATENT APPLICATION

(11) **EP 2 148 203 A1**
(43) Date of publication of application: **27.01.2010**
(21) Application number: 08161031.3
(22) Date of filing: 23.07.2008
(51) Int. Cl.: G01N 33/573

(54) **Azurophilic granule proteases as markers in cardiological diseases**

(71) Applicant: BRAHMS Aktiengesellschaft, 16761 Hennigsdorf (DE)
(72) Inventor: The designation of the inventor has not yet been filed
(74) Representative: Kilger, Ute

(57) **Abstract**

The present invention is concerned with an assay for detecting and/or determining the level of a protease from azurophilic granules of polymorphonuclear neutrophils or a complex thereof with an endogenous inhibitor for risk stratification and/or prognosis and/or therapy control and/or monitoring of a patient having a cardialogical disease or condition.

## Description

The present invention is concerned with an assay for detecting and/or determining the level of a protease from azurophilic granules of polymorphonuclear neutrophils or a complex thereof with an endogenous inhibitor for risk stratification and/or prognosis and/or therapy control and/or monitoring of a patient having a cardiological disease or condition.

Atherosclerosis is associated with inflammatory changes in the blood vessel wall (Libby et al. Circulation 2005, 111, 3481-3488), including macrophage and lymphocyte infiltration. There is an association between neutrophil count and the risk of future acute coronary syndromes (Friedman et al., N. Engl. J. Med. 1974; 290, 1275-8). Recent evidence has documented elevated neutrophil activity in acute coronary syndromes (Naruko et al., Circulation 2002, 106, 2894-900; Buffon et al., N. Engl. J. Med. 2002, 347, 5-12).

Myeloperoxidase (MPO), an enzyme present within the azurophilic granules of neutrophils, may stimulate plaque destabilisation by activation of metalloproteinases (Fu et al., J Biol Chem 2001, 276, 41279-87). Plasma levels of MPO are elevated in coronary artery disease (Zhang et al., JAMA 2001, 286, 2136-42), and predict outcome of patients presenting with chest pain (Brennan et al., N Engl J Med 2003, 349,1595-604) and acute coronary syndromes (Baldus et al., Circulation 2003, 108, 1440-5). Proteinase 3 (PR3) is another major component of neutrophil azurophilic granules, together with other serine proteases such as elastase and cathepsin G (Pham et al., Nat Rev Immunol 2006, 6, 541-50). Azurophilic granules are released in response to powerful stimuli to neutrophils, whereas secretory granules (which also contain PR3 (Witko-Sarsat et al., Blood 1999, 94, 2487-96)) are released with weaker stimuli. PR3 may also be expressed on endothelial cells (Mayet et al., Blood 1993, 82, 1221-9). Neutrophil serine proteases may degrade extracellular matrix; in addition, PR3 has other deleterious effects which may be relevant to a role in pathogenesis of vasculitis in Wegener's granulomatosis. These include induction of apoptosis through a caspase-like activity on endothelial cells (Pendergraft et al., Kidney Int. 2004, Jan, 65(1), 75-84), releasing activated TNFα from its nascent membrane bound precursor form (Robache-Gallea et al., J Biol Chem. 1995, 270, 23688-92; Coeshott et al., PNAS 1999, 96, 6261-6), activation of the pro-inflammatory mediators interleukin-1β (Coeshott et al., PNAS 1999, 96, 6261-6) and interleukin-18 (Sugawara et al., J Immunol. 2001 Dec 1, 167(11), 6568-75) and generation of angiotensin I and II from angiotensinogen (Ramaha et al., Arch Biochem Biophys. 2002, 397, 77-83).
PR3 is rapidly inactivated by irreversible binding to α-1-antitrypsin (SERPIN A1) (Baslund et al., J Immunol Methods 1994, 175, 215-25; Duranton et al., Am J Respir Cell Mol Biol. 2003, 29, 57-61) where the serine protease reacts with the reactive centre loop of the serpin, generating a covalently bound intermediate resulting in the distortion and inactivation of the protease (Stratikos et al., PNAS, 1997, 94, 453-8). Thus, little free PR3 exists in plasma and it is mainly present as a PR3-SERPIN A1 complex (Baslund et al., J Immunol Methods 1994, 175, 215-25).

The source of circulating PR3 may be partly from degranulating, activated neutrophils, being present in both azurophilic and secretory granules. Neutrophils are known to infiltrate myocardium after infarction. Alternatively, or additionally, PR3 may be secreted from endothelial cells (Mayet et al., Blood 1993, 82, 1221-9) or from lung parenchymal cells (Brockmann et al., Arthritis Res. 2002, 4, 220-5). Pneumocytes in normal lung have been demonstrated to express PR3 (Brockmann et al., Arthritis Res. 2002, 4, 220-5), although expression is greatly upregulated in Wegener's Granulomatosis. Active PR3 is rapidly inactivated in the plasma by SERPIN A1 and alpha2-macroglobulin (Pham et al., Nat Rev Immunol 2006, 6, 541-50).

The damaging role of PR3 on cells in the post AMI setting has been shown, such as its pro-apoptotic activity (Pendergraft et al., 2004 Jan, 65(1), 75-84) and its pro-inflammatory effect, by releasing the active forms of TNFα, interleukin-1β, interleukin-18 (Robache-Gallea et al., J Biol Chem. 1995, 270, 23688-92; Coeshott et al., PNAS 1999, 96, 6261-6; Sugawara et al., J Immunol. 2001 Dec 1, 167(11), 6568-75) and the angiotensins (Ramaha et al., Arch Biochem Biophys. 2002, 397, 77-83). These cytokines may be especially relevant to development of heart failure post-AMI, which still occurs despite therapy with inhibitors of the renin-angiotensin system. The involvement of such serine proteases in the development of heart failure is being discussed. For example, there is preliminary evidence that elafin, a serine protease inhibitor, could reduce the vascular and myocardial damage in experimental models of cardiac transplantation (Cowan et al., J Clin Invest. 1996, 97, 2452-68).

According to the present invention it has been surprisingly found that proteases from azurophilic granules of polymorphonuclear neutrophils or a complex thereof with an endogenous inhibitors may be used as an biomarker in cardiological diseases or conditions.

Thus, the present invention is concerned with an assay for detecting and/or determining the level of a protease from azurophilic granules of polymorphonuclear neutrophils or a complex thereof with an endogenous inhibitor as markers in cardiological diseases. The evaluation of the analyte levels measured by the assay may be done independent of established conventional risk factors and also other biomarkers. However, such conventional risk factors and/or other biomarkers may be used in combination with the assay or method or use according to the present invention. The markers provide complementary independent prediction, and the identification of such high risk patients may improve their subsequent management.

This assay provides information for the prediction of adverse outcomes, such as death and heart failure, which may be applied independently of or in combination with established conventional risk factors such as renal function and clinical demographic information.

The measurement of such a protease from azurophilic granules of polymorphonuclear neutrophils or a complex thereof with an endogenous inhibitor provides prognostic information on heart failure and mortality post-AMI, used alone or in conjunction with recognised markers of prognosis such as B-type natriuretic peptide or its precursor (N-terminal proB-type natriuretic peptide or NTproBNP), aminoterminal proANP, proAdrenomedullin or fragments thereof, proEndothelin or fragments thereof, proVasopressin or fragments thereof.

An embodiment of the present invention is a method for risk stratification and/or prognosis and/or therapy control and/or monitoring of a patient having a cardiological disease or condition, comprising:
a) providing a sample from said patient with a cardiological disease or condition,
b) determining the level of a protease from azurophilic granules of polymorphonuclear neutrophils or a complex thereof with an endogenous inhibitor in said sample,
c) attributing the level of said protease from azurophilic granules of polymorphonuclear neutrophils or a complex thereof with an endogenous inhibitor to a prognosis or the degree of severity of the disease or condition or the risk of an adverse outcome for said patient.

In a preferred embodiment of the method according to the present invention, the level of a protease selected from elastase, cathepsin G, proteinase 3 (Pr3) or a complex thereof with an endogenous inhibitor is determined in step b) and attributed to a prognosis or the degree of severity of the disease or condition or the risk of an adverse outcome for said patient in step c).

In a more preferred embodiment of the method according to the present invention, the level of proteinase 3 or the proteinase 3/Serpin A1 complex is determined in step b) and attributed to a prognosis or the degree of severity of the disease or condition or the risk of an adverse outcome for said patient in step c).

In the method according to the present invention, even more preferably the level of the proteinase 3/Serpin A1 complex is determined in step b) and attributed to a prognosis or the degree of severity of the disease or condition or the risk of an adverse outcome for said patient in step c).

In another preferred embodiment of the method according to the present invention, the cardiological disease or condition is selected from the group comprising acute coronary syndrome, including unstable angina (UA), non-ST segment elevation myocardial infarction (NSTEMI) or ST segment elevation myocardial infarction (STEMI).

In the present invention the cardiological disease or condition is preferably acute coronary syndrome. More preferably, the cardiological disease or condition is acute myocardial infarction.

The diagnostic assay used in the context of the present invention can be of any type applied in the field of diagnostics, including but not restricted to assays methods based on enzymatic reactions, luminescence, in particular fluorescence or radiochemicals. The preferred detection methods comprise point-of-care-tests, radioimmunoassays, chemiluminescence- and fluorescence- immunoassays, immunoblot assays, enzyme-linked immunoassays (ELISA), luminex-based bead arrays, and protein microarray assays. The assay types can further be microtitre plate-based, chip-based, bead-based, wherein the biomarker proteins can be attached to the surface or in solution. The assays can be homogenous or heterogeneous assays, sandwich assays, competitive and non-competitive assays (The Immunoassay Handbook, Ed. David Wild, Elsevier LTD, Oxford; 3rd ed. (May 2005), ISBN-13: 978-0080445267; Hultschig C et al., Curr Opin Chem Biol. 2006 Feb;10(1):4-10. PMID: 16376134)

In another preferred embodiment of the method according to the present invention, in step b) the level of said protease or a complex thereof with an endogenous inhibitor in said sample is determined with a diagnostic assay.

In another preferred embodiment of the method according to the present invention, the protease from azurophilic granules of polymorphonuclear neutrophils or a complex thereof with an endogenous inhibitor is detected by use of two capture molecules, wherein the two capture molecules may be added to the sample sequentially or simultaneously.

A nonlimiting Example of the method according to the present invention comprises in step b) the further steps of b1) adding said sample to an assay comprising one or more first capture molecules directed against said protease or said endogenous inhibitor or said complex of the protease with an endogenous inhibitor, and further b2) adding one or more second capture molecules directed against said protease or said endogenous inhibitor or said complex of the protease with an endogenous inhibitor to said assay, whereby the first capture molecule and the second capture molecule may not be both directed against the inhibitor, wherein the steps b1) and b2) may be executed sequentially or simultaneously.

In a particularly preferred embodiment the assay comprises two capture molecules, even more preferably antibodies which are both present as dispersions in a liquid reaction mixture, wherein a first marking component is attached to the first capture molecule, wherein said first marking component is part of a marking system based on fluorescence- or chemiluminescence-quenching or amplification, and a second marking component of said marking system is attached to the second capture molecule, so that upon binding of both capture molecules to said protease or said endogenous inhibitor or said complex of the protease with an endogenous inhibitor, a measurable signal is generated that allows for the detection of the formed sandwich complexes in the solution comprising the sample.

Even more preferred, said marking system comprises rare earth cryptates or rare earth chelates in combination with a fluorescence dye or chemiluminescence dye, in particular a dye of the cyanine type. Most preferred, said marking system is the system applied in the Kryptor^{®} technology by BRAHMS AG.

In another even more preferred embodiment of the method according to the present invention, step c) comprises:
c1)measuring a signal corresponding to the amount of bound protease or bound complex thereof with an endogenous inhibitor,
c2) comparing the intensity of said signal of step c1) with pre-recorded data, wherein said pre-recorded data correlates levels of the protease or complex thereof with an endogenous inhibitor with a prognosis or the degree of severity of the disease or condition or the risk of an adverse outcome.

In another preferred embodiment of the method according to the present invention, the level of the protease or complex thereof with an endogenous inhibitor is correlated with a prognosis or the degree of severity of the disease or condition or the risk of an adverse outcome by a method selected from the group comprising correlation with respect to the median of the level of the protease or complex thereof with an endogenous inhibitor in an ensemble of pre-determined samples; correlation with respect to quantiles (e.g. quartiles or percentiles) of the level of the protease or complex thereof with an endogenous inhibitor in an ensemble of pre-determined samples; or correlation with a mathematical model including the level of the protease or complex thereof with an endogenous inhibitor, preferably Cox Regression.

In another preferred embodiment of the method according to the present invention, said sample is a plasma sample, a serum sample, a blood sample or fractions thereof, a lymphatic fluid sample, a urine sample or an extract of any of the aforementioned samples.

In one particular embodiment of the invention, said signal is generated by a label bound to said second capture molecule.

In another particular embodiment of the invention, said signal is generated by a label bound to a third capture molecule, wherein said third capture molecule is specific for said second capture molecule, and wherein said third capture molecule is added to said assay before step c1). In the method according to the present invention, the third capture molecule may be added after the second capture molecule, or simultaneously with the second capture molecule.

In another preferred embodiment of the method according to the present invention, additionally the level of one or more further markers or clinical parameters, which are associated with or useful for risk stratification and/or prognosis and/or therapy control and/or monitoring of a patient having a cardiological disease or condition is determined.

In another more preferred embodiment of the method according to the present invention, the clinical parameter is a parameter selected from the group comprising age, gender, prior history of diseases, in particular myocardial infarction, Angina Pectoris, hypertension, Diabetes mellitus, hypercholesterolaemia, ST-elevation AMI, body mass index, genetic predisposition / family history, ethnic background, habits which affect said propensity, such as smoking, alcohol consumption, diet, or parameters upon hospitalization, such as Killip Class > 1, or thrombolytic medication upon hospitalization.

In another more preferred embodiment of the method according to the present invention, the further markers are selected from the group comprising proBNP or fragments thereof of at least 12 amino acids including BNP and NT-proBNP, proANP or fragments thereof of at least 12 amino acids including NT-proANP and MR-proANP, proAdrenomedullin or fragments thereof of at least 12 amino acids including Adrenomedullin, PAMP and MR-proADM, proEndothelin or fragments thereof of at least 12 amino acids including Endothelin-1, big-Endothelin-1, CT-proET-1 and NT-proET-1, proVasopressin or fragments thereof of at least 8 amino acids including Vasopressin, Copeptin and Neurophysin 2, myeloperoxidase, Troponin, FABP, C-reactive protein, procalcitonin, interleukin-6, ST-2, GDF-15, ischemia modified albumin, or fragments thereof.

In another even more preferred embodiment of the method according to the present invention, the further marker is NT-proBNP or a fragment thereof of at least 12 amino acids.

Another embodiment of the present invention is the use of a kit comprising at least one capture molecule directed against a protease from azurophilic granules of polymorphonuclear neutrophils and/or a complex thereof with an endogenous inhibitor for detecting and/or determining the level of said protease from azurophilic granules of polymorphonuclear neutrophils and/or a complex thereof with an endogenous inhibitor in said sample for risk stratification and/or therapy control and/or monitoring and or prognosis of a patient having a cardiological disease or condition.

Preferably, the kit according to the present invention comprises one or more first capture molecules directed against a protease from azurophilic granules of polymorphonuclear neutrophils or against an endogenous inhibitor of said protease or against a complex of said protease with said endogenous inhibitor, and comprises one or more second capture molecules directed against a protease from azurophilic granules of polymorphonuclear neutrophils or against an endogenous inhibitor of said protease or against a complex of said protease with said endogenous inhibitor.
More preferably, said first capture molecule and said second capture molecule may not both be directed against said endogenous inhibitor

More preferably, the kit according to the present invention comprises first and second capture molecules directed against a protease selected from elastase, cathepsin G, proteinase 3 (Pr3) and against an endogenous inhibitor of said protease, respectively.

Even more preferably, the kit according to the present invention comprises capture molecules directed against proteinase 3 and Serpin A1, respectively.

Most preferably, the kit according to the present invention comprises capture molecules directed against the complex of proteinase 3 and Serpin A1.

In another particularly preferred embodiment of the use of a kit according to the present invention, the prognosis or the degree of severity or the disease or condition or the risk of an adverse outcome is determined.

In a particularly preferred embodiment of the method or the use of a kit according to the present invention, said adverse outcome is death or heart failure.

Preferably, the use of the kit according to the present invention is done as described above in the embodiments of the method of the present invention.

In the context of the present invention, capture molecules may be selected from the group comprising a nucleic acid molecule, a carbohydrate molecule, a PNA molecule, a protein, an antibody, a peptide or a glycoprotein. Preferably, the capture molecules are antibodies, including fragments thereof with sufficient specificity to a target, and including recombinant antibodies, as well as chemically and/or biochemically modified derivatives of said antibodies or fragments derived from the variant chain with a length of at least 12 amino acids thereof.

In the context of the present invention, dyes of the cyanine type, also termed cyanine dyes, may be streptocyanines, also termed open chain cyanines of the general chemical structure R₂N⁺=CH(CH=CH)ₙ-NR₂, hemicyanines of the general chemical structure aryl=N⁺=CH(CH=CH)ₙ-NR₂ or closed chain cyanines of the general chemical structure aryl=N⁺=CH(CH=CH)n-N=aryl, whereby R independently specifies an alkyl group which may be substituted by one or more substituents and whereby aryl independently specifies an aryl or heteroaryl group which may be substituted by one or more substituents. As such cyanine dyes are well known and many derivatives are commercially available, the skilled person will know which of the se well known dyes to choose for a given purpose. Thus, a detailed description of these dyes will not be given in the present specification; a more detailed description, including examples of cyanine dyes can be found in: http://en.wikipedia.org/wiki/Cyanine and: Kirk-Othmer, Encyclopedia of chemical technology, 4th ed., executive editor, J. I. Kroschwitz; editor, M. Howe-Grant, John Wiley & Sons, 1993, vol. 7, p. 782-809. Specific examples of cyanine dyes used in the present invention are Cy 3 and Cy 5.

In the context of the present invention, a signal is the presence or absence of a detectable response, ergo one lying within the sensitivity range of device used to detect said response, which specifically can be attributed to the presence of a complex between the analyte and the capture probe or probe. Said signal may be attributed to various types of effects which are commonly exploited in methods for the detection of analytes, such as for instance the absorption, emission or quenching of electromagnetic radiation, wherein electromagnetic radiation may be fluorescence, luminescence, visible light, UV and IR, or to radioactivity, magnetism or nuclear or electronic spin. In the present invention, the above-mentioned analyte is a protease from azurophilic granules of polymorphonuclear neutrophils or a complex thereof with an endogenous inhibitor.

In the present invention, the term risk stratification denotes an assignment of a probability to experience certain adverse events to an individual.

In the present invention, the term prognosis denotes a prediction of how a subject's (e.g. a patient's) medical condition will progress. This may include an estimation of the chance of recovery or the chance of an adverse outcome for said subject.

In the present invention, the term monitoring denotes the observation of the state or progression of a subject's medical condition by measuring the level of a certain diagnostic marker or markers for said medical condition at various points of time.

In the present invention, the term therapy control denotes the attribution of a certain form of treatment, such as the administration of a medicament, to the state or progression of a subject's medical condition by measuring the level of a certain diagnostic marker or markers for said medical condition at various points of time, preferably before and after the treatment. In this way, it may be determined whether said treatment is adequate to treat said medical condition, or whether the therapy will have to be adjusted, e.g. by altering the dosage of the medicament, or will have to be replaced by another form of treatment, e.g. another medicament or another type of therapeutic action.

### Brief description of the figures

Figure 1: Kaplan-Meier analysis showing time to adverse events (death or heart failure) in patients stratified by PR3-SERPIN A1.
Figure 2: Kaplan-Meier analysis showing time to adverse events (death or heart failure) in patients stratified according to whether both PR3-SERPIN A1 and NTproBNP are below their respective median values, either marker being elevated, or both markers elevated above median.
Figures 3 to 7 show the results of logistic regression analyses of patient data:

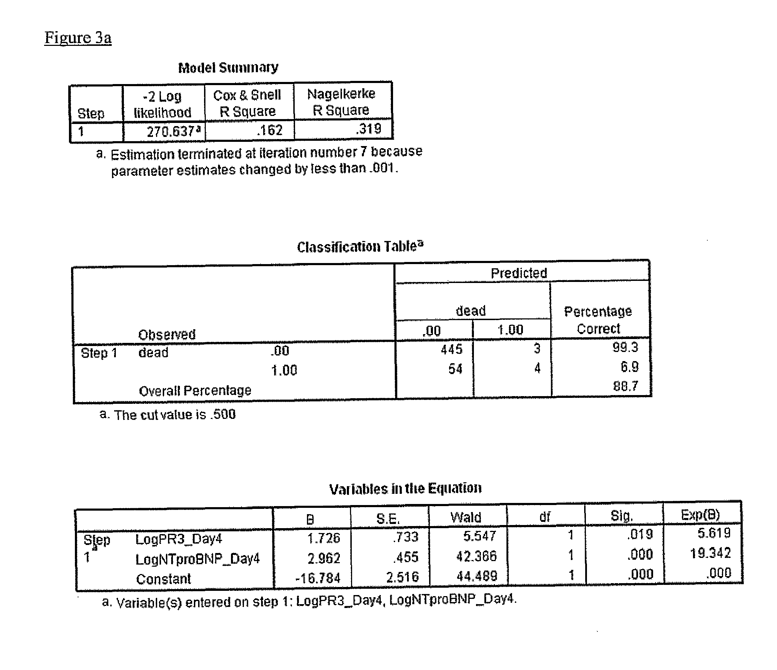

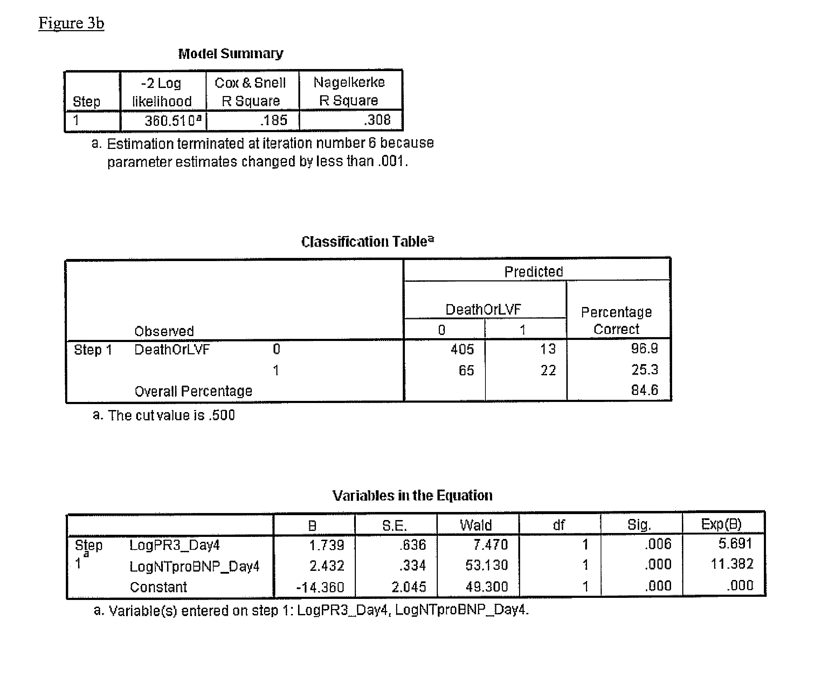

Figure 3 a: Predictors of Death for NTproBNP and PR3 on Day 4
Figure 3b: Predictors of Death and Heart Failure for NTproBNP and PR3 on Day 4

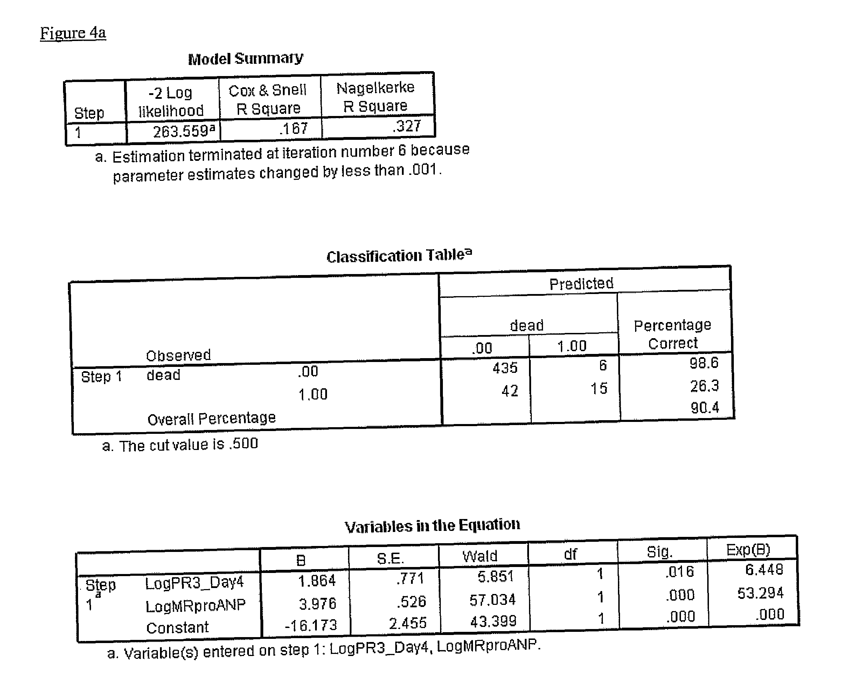

Figure 4a: Death Endpoint for MRproANP with PR3

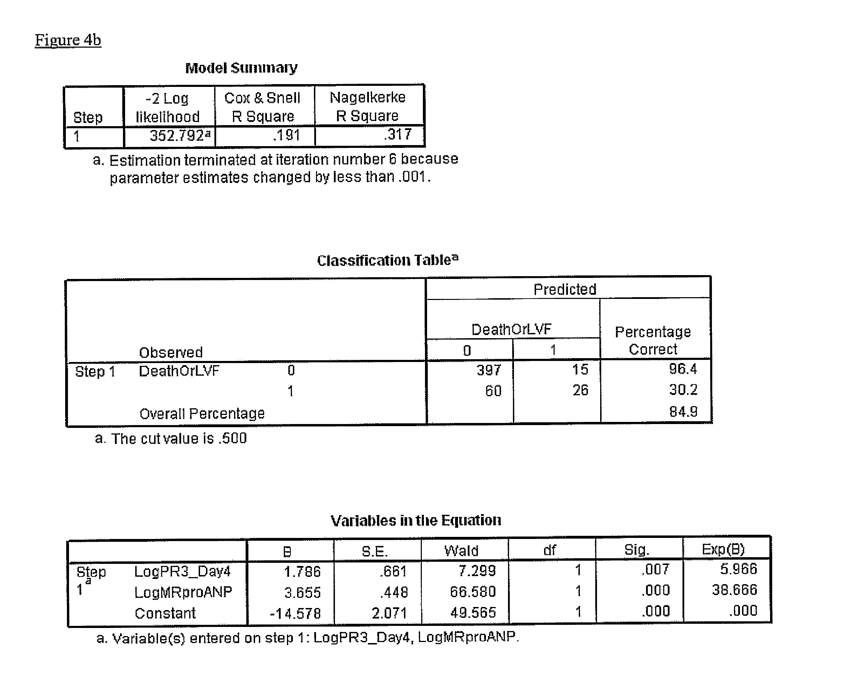

Figure 4b: Death and Heart Failure Endpoint for MRproANP with PR3

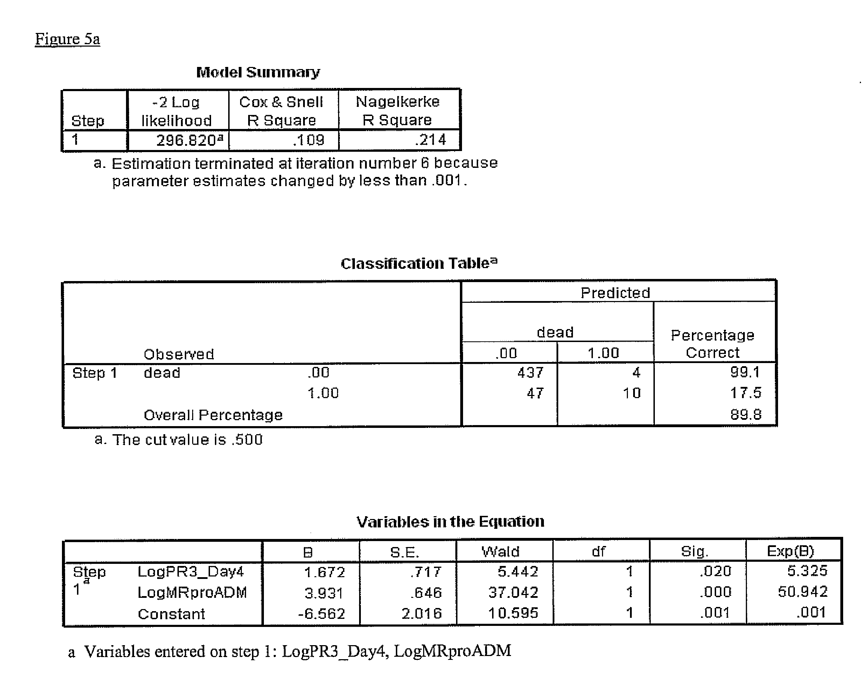

Figure 5a: Death Endpoint for MRproADM with PR3

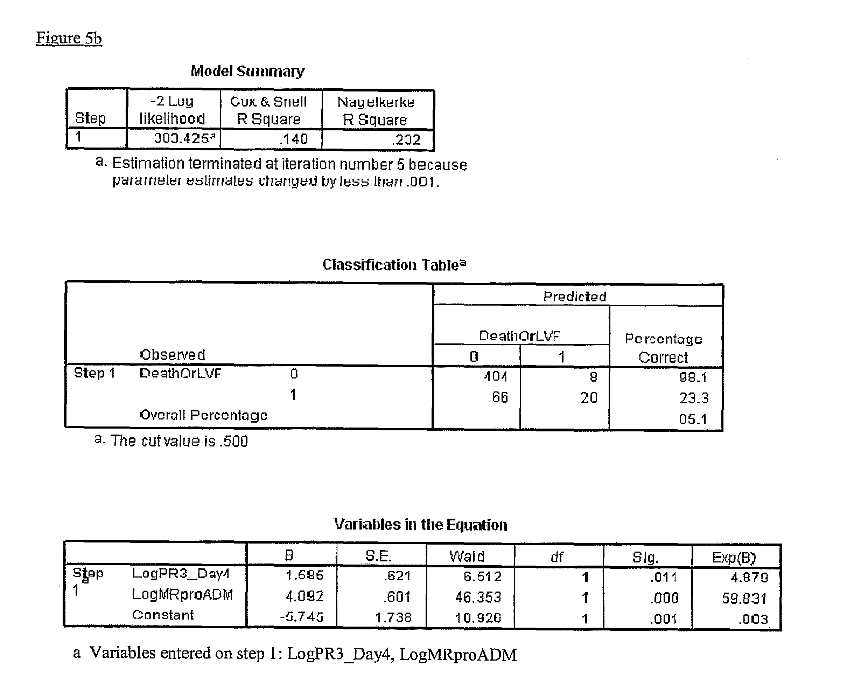

Figure 5b: Death and Heart Failure Endpoint for MRproADM with PR3

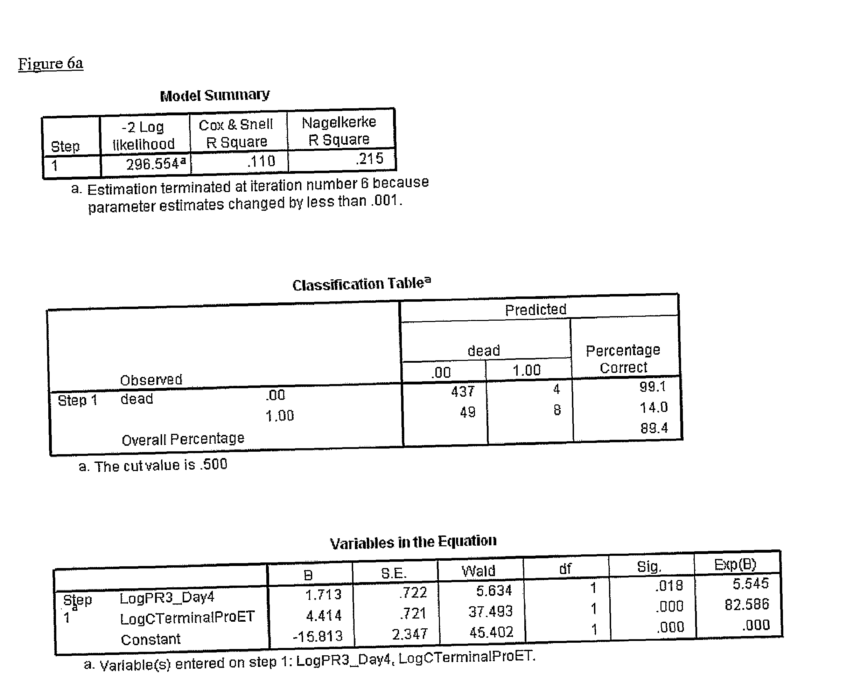

Figure 6a: Death Endpoint for C-terminal proET with PR3

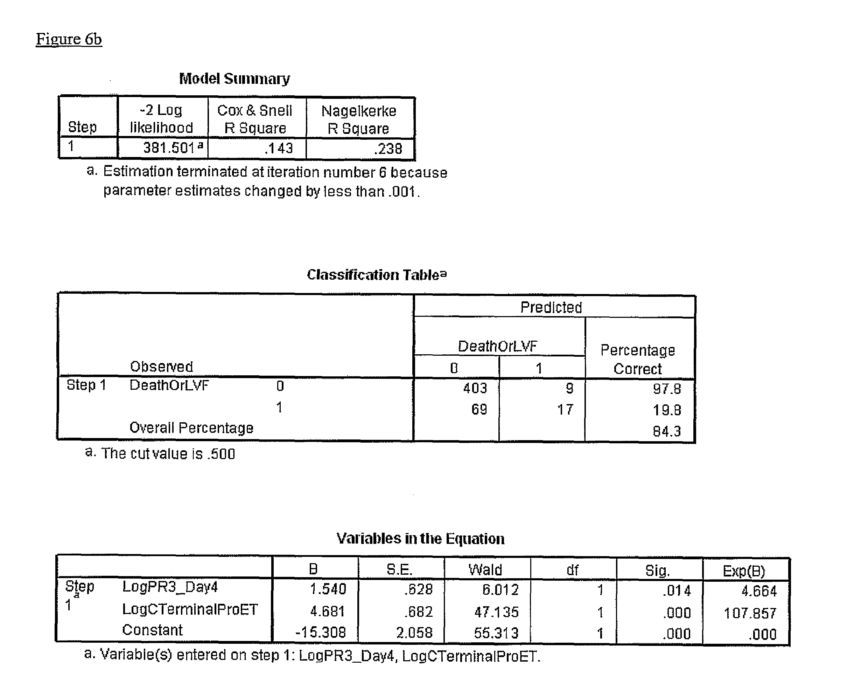

Figure 6b: Death and Heart Failure Endpoint for C-terminal proET with PR3

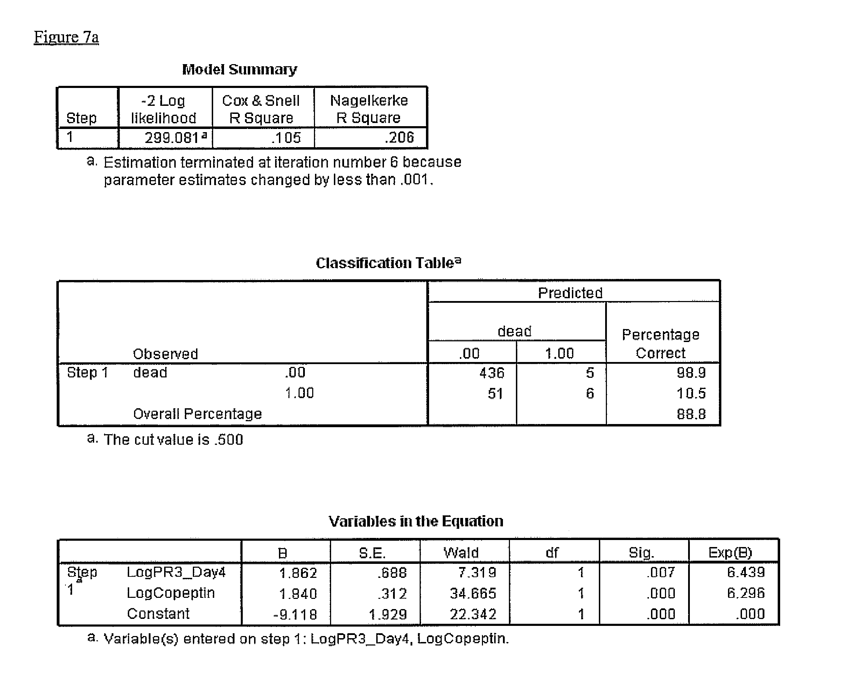

Figure 7a: Death Endpoint for Copeptin with PR3

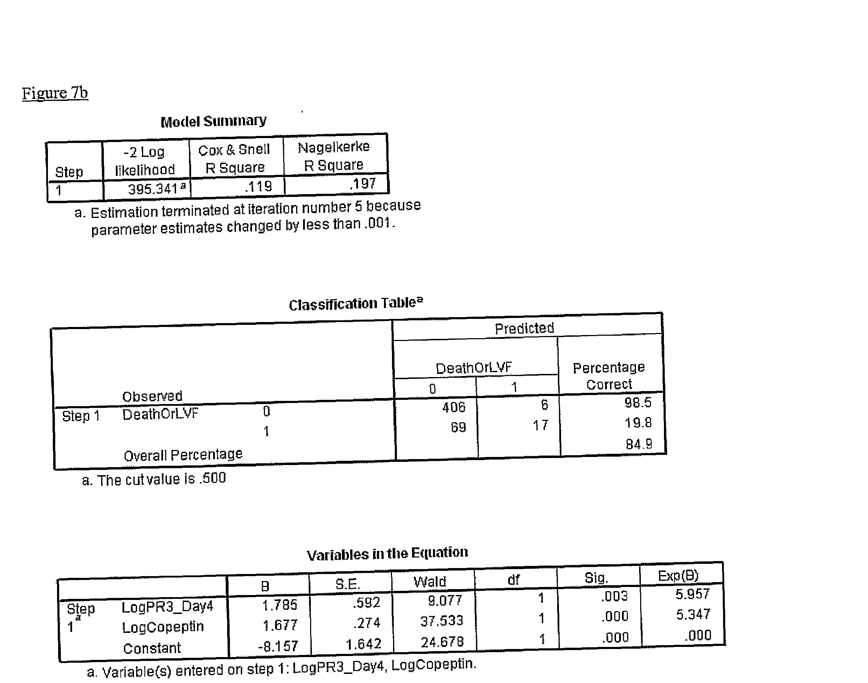

Figure 7b: Death and Heart Failure Endpoint for Copeptin with PR3

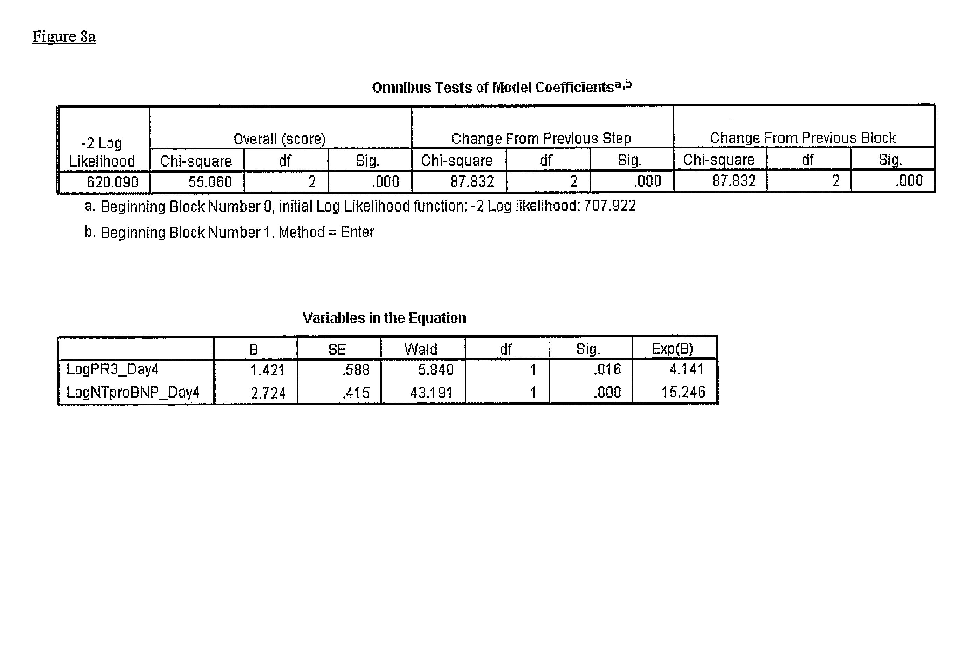

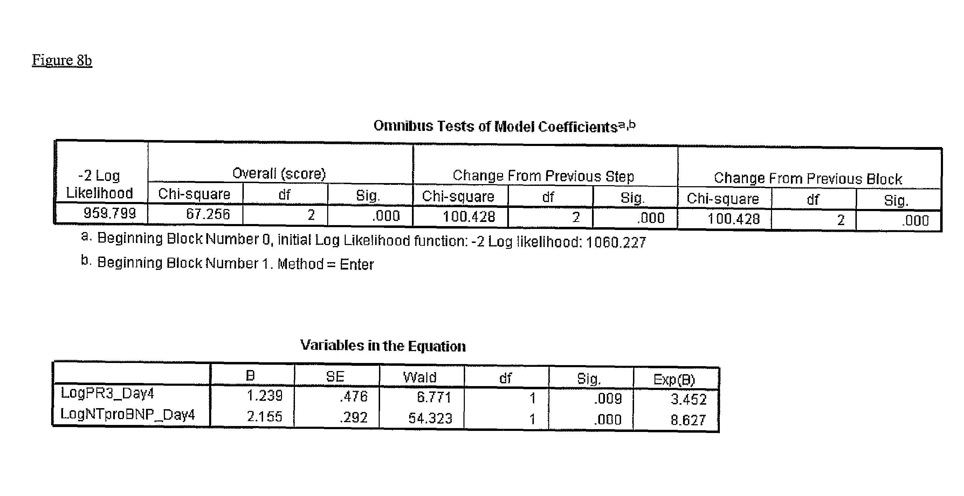

Figures 8 to 12 show the results of Cox proportional hazard analyses of patient data
Figure 8a: Predictors of Death for NTproBNP and PR3 on Day 4
Figure 8b: Predictors of Death and Heart Failure for NTproBNP and PR3 on Day 4

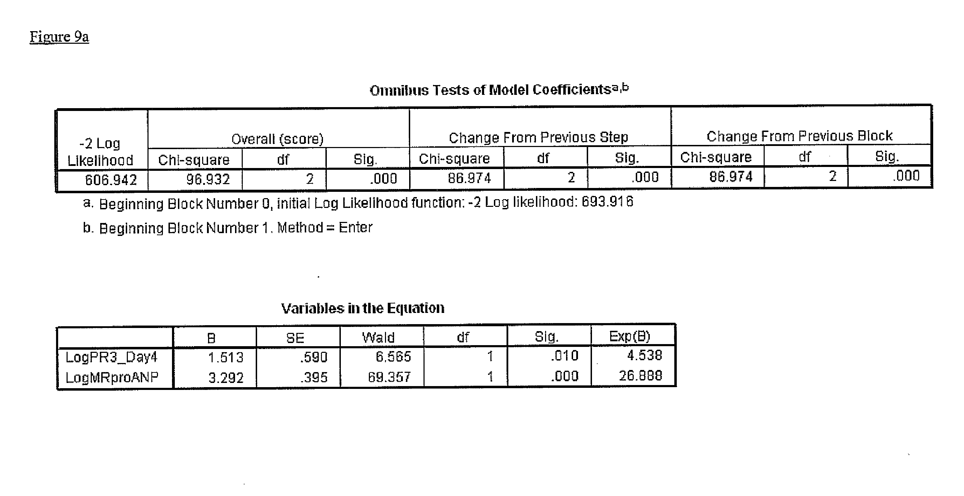

Figure 9a: Death Endpoint for MRproANP with PR3

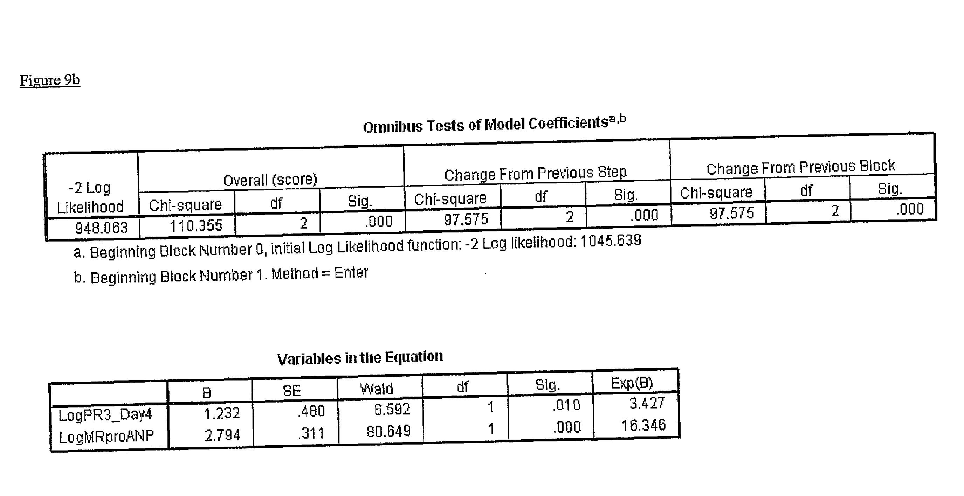

Figure 9b: Death and Heart Failure Endpoint for MRproANP with

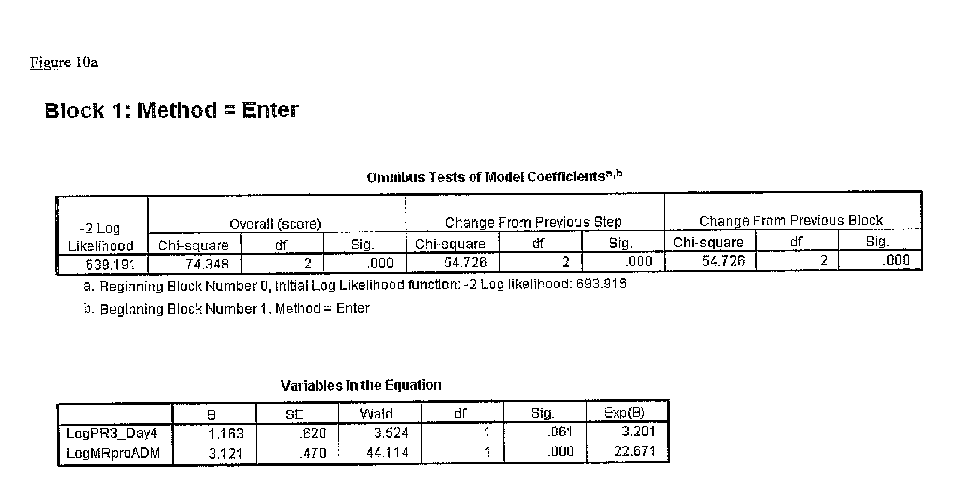

Figure 10a: Death Endpoint for MRproADM with PR3

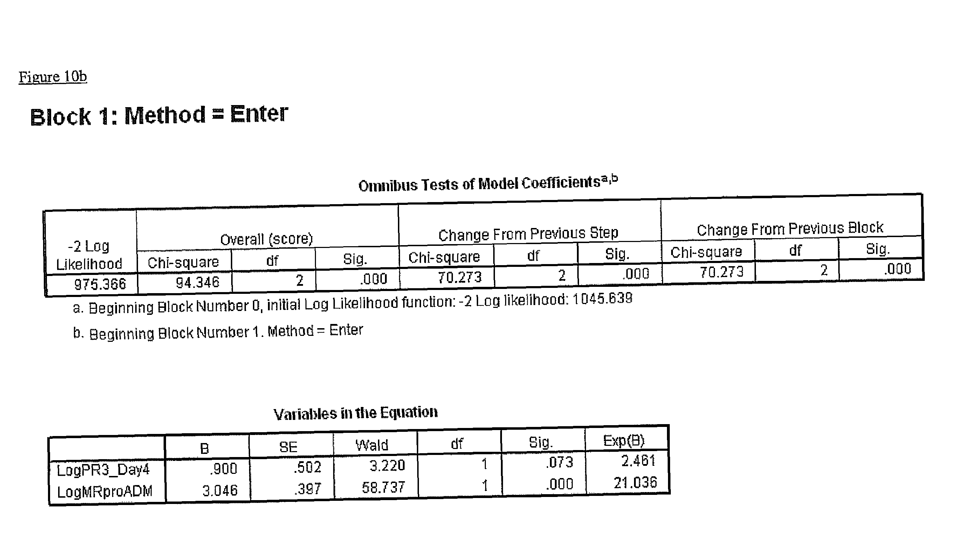

Figure 10b: Death and Heart Failure Endpoint for MRproADM with PR3

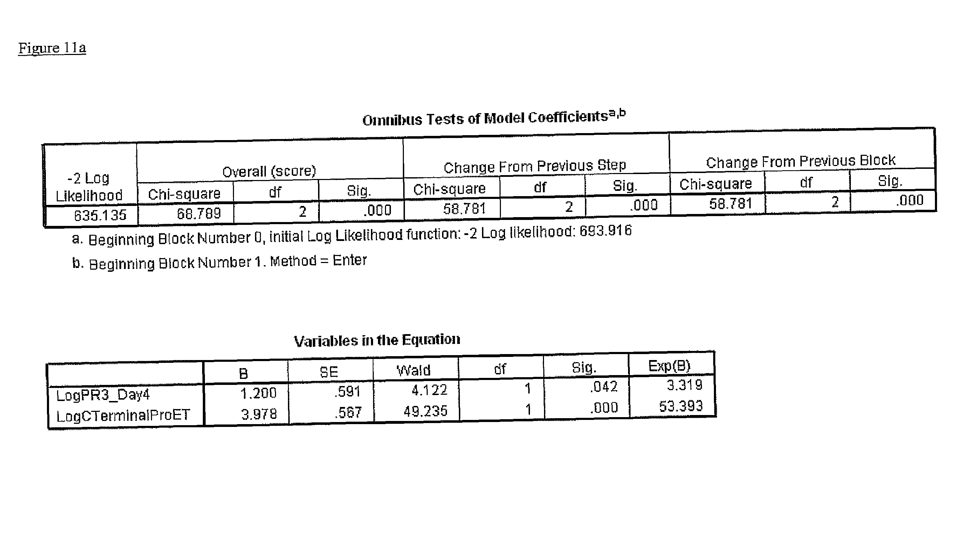

Figure 11a: Death Endpoint for C-terminal proET with PR3

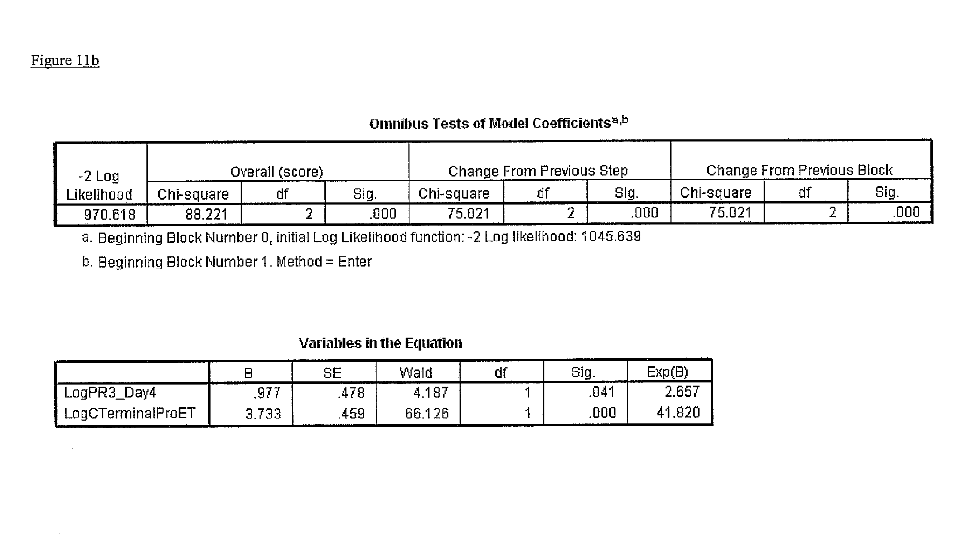

Figure 11b: Death and Heart Failure Endpoint for C-terminal proET with PR3

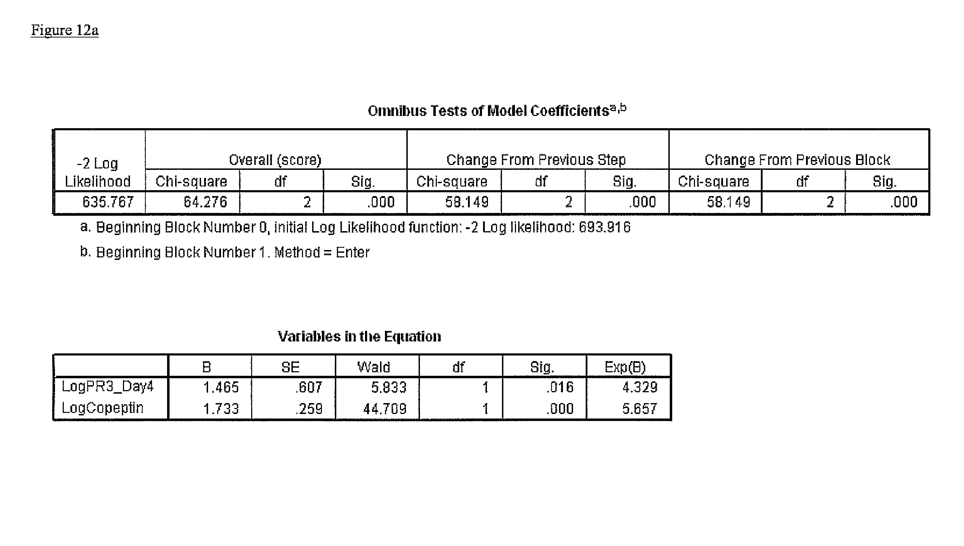

Figure 12a: Death Endpoint for Copeptin with PR3

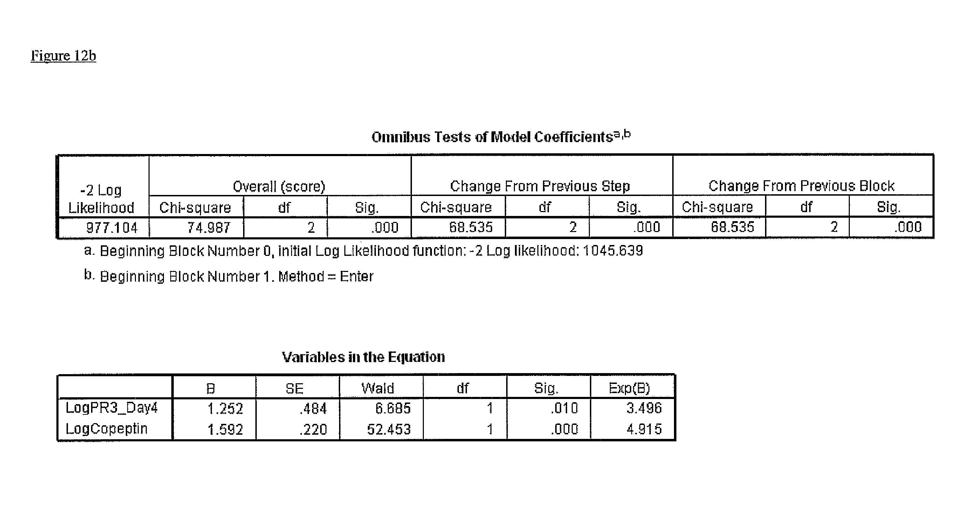

Figure 12b: Death and Heart Failure Endpoint for Copeptin with PR3

### EXAMPLES

900 consecutive patients with acute myocardial infarction were recruited. Written informed consent was obtained from patients and the study complied with the Declaration of Helsinki and was approved by the local ethics committee. AMI was defined with at least two of three standard criteria at presentation, i.e. appropriate symptoms, acute ECG changes of infarction (ST elevation or depression, new left bundle branch block) and a rise in troponin T above the 99^{th} percentile for our population. Exclusion criteria included malignancy or recent surgery (within one month).

**Table 1: Characteristics of patients in the study. Values are means (SD) or numbers (%)**

| | Patients | | Patients |
|---|---|---|---|
| Number (male) | 900 (681)? | Territory of Infarct | |
| Age (in years) | 64.6 ± 12.4 | Anterior | 369 (41.0) |
| Previous Medical History | | Inferior | 373 (41.4) |
| Myocardial infarction | 165 (18.3) | Other/undetermined | 158 (17.6) |
| Angina Pectoris | 195 (21.7) | Killip Class on Admission | |
| Hypertension | 391 (43.4) | I | 457 (50.7) |
| Diabetes mellitus | 192 (21.3) | II | 357 (39.7) |
| Hypercholesterolaemia | 221 (24.6) | III | 77 (8.6) |
| Current/Ex-Smokers | 560 (62.2) | IV | 9 (1.0) |
| ST-elevation AMI | 777 (86.3) | Peak CK (IU/L) | 1157 ± 1263 |
| Thrombolytic | 529 (58.8) | Creatinine (µmol/L) | 103.9 ± 35.4 |

### Plasma samples:

Blood samples were drawn at 2-5 days after the onset of chest pain for determination of plasma PR3-SERPIN A1 and NTproBNP. Samples of day 4 were used to measure MR-proANP, MR-proADM, CT-proET-1 and Copeptin. After 15 minutes bed rest, 20mL blood was collected into tubes containing EDTA and aprotinin. All plasma was stored at -80°C until assayed, blind to clinical details, in a single batch.

### Echocardiography:

Transthoracic echocardiography was performed in patients using a Sonos 5500 instrument (Philips Medical Systems, Reigate, UK). A 16-segment left ventricular wall motion index (LVWMI) based on the American Society of Echocardiography mode was derived by scoring each LV segment (1=normal, 2=hypokinesis, 3=akinesis and 4=dyskinesis (Paradoxical Motion)), and dividing the total by the number of segments scored. Left ventricular ejection fraction (LVEF) was calculated using the biplane method of discs formula (Schiller et al., J Am Soc Echocardiogr 1989, 2, 358-67). Impaired LV systolic function was defined as an EF<40% or a LVWMI > 1.8.

### NTproBNP assay:

Plasma NTproBNP assay was measured using a non-competitive immunoluminometric assay as previously published (Omland et al. Circulation 2002, 106, 2913-8). Samples or NTproBNP standards (10µL) were incubated in ELISA plate wells coated with mouse monoclonal IgG directed to the C-terminal of NTproBNP. Detection was with biotinylated rabbit N-terminal antibody followed by methyl-acridinium ester (MAE)-labelled streptavidin on a MLX plate luminometer (Dynex Technologies Ltd., Worthing, UK). The lower limit of detection was 0.3 pmol/L. This highly specific assay shows no cross-reactivity with atrial natriuretic peptide, BNP, or C-type natriuretic peptide.

### PR3-SERPIN A1 assay:

Plasma PR3-SERPIN A1 was measured using a non-competitive immuno-luminometric assay modified from a previously published method (Baslund et al., J Immunol Methods 1994, 175, 215-25). ELISA plate wells were coated with 200ng of mouse monoclonal antibody to PR3 (clone 1B10, IgG₂ₐ, HyTest Ltd., Turku, Finland). Standards were produced by incubating PR3 with a 20 fold molar excess of SERPIN A1 (both from Sigma Aldrich Co. Ltd., Gillingham, UK). Samples or standards (10 µL) were incubated with assay buffer (100µL) in the coated wells for 24 h at 4°C. Following washes, a specific SERPIN A1 rabbit antibody (Dako UK Ltd., Ely, UK) was pipetted into the wells (20 ng/100 µL) and incubated for 3 h with shaking at 250 Hz at room temperature. Following washes, a goat biotinylated anti-rabbit IgG (Rockland Immunochemicals Inc., Gilbertsville, PA, USA, previously pre-adsorbed with human, rabbit, mouse serum proteins) at a dilution of 1:100000 was incubated within the wells for 1 h, followed by MAE-labelled streptavidin for another 1,5 hours. Chemiluminescence was elicited with sequential injections of H₂O₂ in nitric acid, followed by sodium hydroxide containing cetyl trimethylammonium bromide, as described (Omland et al. Circulation 2002, 106, 2913-8). Intra and inter-assay coefficients of variation were found to be less than 10%.

### Comparative Myeloperoxidase (MPO) assay:

The myeloperoxidase (MPO) assay based on a 2 site non-competitive immuno-luminometric assay and employed an ELISA plate immobilised monoclonal MPO antibody (100 ng/100 µL, Research Diagnostics Inc., New Jersey, USA) and a rabbit polyclonal antibody (50 ng/100 µL, Merck BioSciences, Nottingham, UK), as previously described (Ng et al., Am Heart J. 2006, 152, 94-101). Standards and 10 µL of plasma were incubated in plates overnight, before washing and detection using goat biotinylated anti-rabbit IgG and streptavidin-MAE, as described for the PR3-SERPIN A1 assay. The MPO assays had inter- and intra-assay coefficients of variation < 10%, with a lower limit of detection of 0.3 ng/ml.

### MR-proANP assay:

MR-proANP was detected using a novel commercial sandwich immunoassay in the chemiluminescence/coated tube-format (BRAHMS AG) as described (Morgenthaler et al., Clin. Chem. 2004 Jan, 50(1), 234-6). Briefly, patient samples (1:40 dilution of 5 µl plasma in incubation buffer) or standards were added in duplicate to antibody-coated tubes (affinity purified sheep polyclonal antibodies directed against proANP peptide 73-90) and incubated for 30 min at room temperature. After five washes with 1 ml washing buffer, 200 µl tracer was added, containing acridinium ester-labelled anti-proANP antibody (affinity purified sheep polyclonal antibodies directed against proANP peptide 53-72), followed by 30 min incubation at room temperature. Tubes were washed three times with 1 ml washing buffer, and detection was performed in a LB952T luminometer (Berthold, Germany; 1 s detection time per sample). Relative light units of the chemiluminescence assay were expressed in pmol/L MR-proANP, as calculated from a calibration curve (4-1800 pmol/l) that was included in every analytical run. The lower detection limit of the assay is 4.3 pmol/l and the functional sensitivity of the assay (inter-assay coefficient of variation < 20 %) is 11 pmol/L MR-proANP.

### MR-proADM assay:

MR-proADM was detected using a novel commercial assay in the chemiluminescence/coated tube-format (BRAHMS AG) to be done as described (Morgenthaler et al., Clin Chem. 2005 Oct, 51(10), 1823-9.). Briefly, tubes were coated with a purified sheep polyclonal antibody raised against a peptide representing amino acids 83-94 of pre-pro-ADM. A purified sheep polyclonal antibody raised against a peptide representing amino acids 68-86 of pre-pro-ADM was labelled with MACN-Acridinium-NHS-Ester (InVent GmbH, Germany) and used as tracer. Dilutions of a peptide representing amino acids 45-92 of pre-pro-ADM in normal horse serum served as standards. The immunoassay was performed by incubating 10 µl of samples/standards and 200 µl tracer in coated tubes for 2 h at room temperature. Tubes were washed 4 times with 1 ml of LIA wash solution (BRAHMS AG), and bound chemiluminescence was measured using a LB952T luminometer (Berthold, Germany).

### CT-proET-1 assay:

CT-proET-1 was detected using a novel commercial assay in the chemiluminescence/coated tube-format (BRAHMS AG), as described (Papassotiriou et al., Clin. Chem. 2006 Jun, 52(6), 1144-51). Briefly, tubes were coated with a purified sheep polyclonal antibody raised against a peptide representing amino acids 168-181 of pre-proET-1. A purified sheep polyclonal antibody raised against a peptide representing amino acids 200-212 of pre-proET-1 was labelled with MACN-Acridinium-NHS-Ester (InVent GmbH, Germany) and used as tracer.

Dilutions of a peptide representing amino acids 169-212 of pre-proET-1 in normal horse serum served as standards. The immunoassay was performed by incubating 50 µl of samples/standards and 200µl tracer in coated tubes for 2 h at room temperature. Tubes were washed 4 times with 1 ml of LIA wash solution (BRAHMS AG), and bound chemiluminescence was measured using a LB952T luminometer (Berthold, Germany).

### Copeptin assay:

Determination of Copeptin (C-terminal proVasopressin) in the chemiluminescence/coated-tube format was performed as described (Morgenthaler et al., Clin Chem. 2006 Jan, 52(1), 112-9). Briefly, the tubes were coated with a purified sheep polyclonal antibody raised against a peptide representing positions 132-147 of pre-pro-Vasopressin. A purified sheep polygonal antibody raised against a peptide representing positions 149-164 of pre-pro-Vasopressin was labelled with MACN-Acridinium-N-hydroxysuccinimide ester and used as tracer. Dilutions of a peptide representing positions 132-164 of pre-pro-AVP in normal horse serum served as standards. The immunoassay was performed by incubating 50 µl of samples/standards and 200 µl tracer in coated tubes for 2 hours at room temperature. The tubes were washed 4 times with 1 ml of LUMItest wash solution (BRAHMS AG, Hennigsdorf Germany), and bound chemiluminescence was measured with an LB952T luminometer (Berthold, Bad Wildbach Germany).

### End points:

The primary endpoint was the combination of all-cause mortality and hospitalisation for heart failure. Secondary endpoints included the occurrence of further myocardial infarction. Hospitalization for heart failure was defined as a hospital admission for which heart failure was the primary reason. There was a minimum 60-day follow-up of all surviving patients.

### Statistical analysis:

Statistical analyses were performed on SPSS Version 14.0 (SPSS Inc, Chicago, Illinois). Continuous variables were compared using the Mann Whitney U test. ANOVA with the Bonferroni correction for multiple comparisons was employed when more than 2 groups were compared. Correlation analysis employed Spearman's rho, and binary logistic regression and Cox proportional hazard analyses were used to develop models in order to test the independent predictive power for factors and variables. Plasma levels of NTproBNP, MPO and PR3-SERPIN A1 were normalised by log transformation. Thus, odds ratios and hazard ratios refer to a tenfold rise in the levels of these markers. Kaplan-Meier survival analysis was used to assess dichotomised values of NTproBNP and PR3-SERPIN A1. Receiver-operating characteristic (ROC) curves were generated and the area under the curves (AUC) was calculated to assess the accuracy of predictors. A p value of less than 0.05 was deemed to be statistically significant.

### Patient characteristics:

The demographic features of the patient population are shown in Table 1. Median length of follow-up was 347 days with a range of 0-764 days. No patient was lost to follow-up. The index AMI was classed as ST-elevation MI (STEMI) in 777 of the patients, 529 (68.1%) of whom received thrombolytic therapy. Echocardiographic data were available for 622 (69.1 %) of the patients during the index admission. During follow-up, 92 (10.2%) of the 900 patients died, 66 (7.3%) experienced readmission with heart failure, 143 (15.9%) patients experienced either end-point and 62 (6.9%) experienced hospitalisation with recurrent AMI.

### Plasma PR3-SERPIN A1, NTproBNP and MPO levels:

Plasma levels of PR3-SERPIN A1 in patients with AMI ranged from 29.3-4035.5 ng/ml with a median of 504.6 ng/ml, and were elevated compared to the established normal range (median [range] 350 [110-580] ng/ml). PR3-SERPIN A1 was significantly higher in patients who died (666.2 [226.8-4035.5] ng/ml, P<0.001 using Bonferroni's correction) or were readmitted with heart failure (598 [231.6-1803.9] ng/ml, P<0.004) compared to event free survivors (486.9 [29.3-3118.2] ng/ml). In contrast, levels of PR3-SERPIN A1 in those readmitted with recurrent AMI (482.5 [168.9-1753.9] ng/ml) were similar to those in event free survivors. PR3-SERPIN A1 levels were similar in males and females, and in those with or without previous histories of hypertension, angina, AMI or heart failure, but were slightly elevated in those with diabetes (547.0 [76.9-4035.5] ng/ml) vs. non-diabetic patients (495.1 [29.3-3118.2] ng/ml, P<0.018).
Plasma PR3-SERPIN A1 levels were modestly correlated with plasma glucose (rₛ= 0.08, p< 0.03), log creatinine (rₛ = 0.13, p< 0.001), Killip class (rₛ = 0.10, p< 0.002), NTproBNP (rₛ =0.25, p< 0.001) and creatine kinase (rₛ =0.13, p< 0.001) but had no relationship to age, site of infarction, presence or absence of ST elevation and whether thrombolysis was administered or not.
In confirmation of previous work, plasma NTproBNP was higher in patients who died (5955.4 [9.02-14057.2] pmol/L) or were readmitted with heart failure (3106.6 [2,4-12282.9] pmol/L) compared to event free survivors (806.8 [0.3-28886] pmol/L; p<0.001 for both). NTproBNP levels were higher in females (1951.7 [22.9-15732] pmol/L) compared to males (871.6 [0.3-28886] pmol/ml; p<0.001) and correlated with age (rₛ= 0.46, p< 0.001), plasma glucose (rₛ= 0.15, p< 0.001), log creatinine (rₛ = 0.27, p< 0.001), Killip class (rₛ = 0.29, p< 0.001) and creatine kinase (rₛ =0.13, p< 0.001)
Plasma MPO was significantly higher in patients who died (36.7 [6.44-132.1] ng/ml, P<0.035 using Bonferroni's correction) compared to event free survivors (24.6 [0.3-405.2] ng/ml). In contrast those who were readmitted with heart failure (27.6 [6.5-210.8] ng/ml) or with recurrent AMI (23.9 [5.3-189.9] ng/ml) had similar values of MPO to event free survivors. MPO levels were higher in females (30.3 [0.3-405.2] ng/ml) compared to males (23.9 [3.6-215.1] ng/ml; p<0.001) and in patients with diabetes (30.4 [5.1-238.7] ng/ml) compared to non-diabetic patients (24.0 [0.3-405.2] ng/ml; p<0.001) and were correlated with age (rₛ= 0.12, p< 0.001), plasma glucose (rₛ= 0.10 p< 0.009, log creatinine (rₛ = 0.11, p< 0.001), and Killip class (rₛ = 0.11, p< 0.002).

There was a modest correlation of Plasma PR3-SERPIN A1 with impaired LV systolic function (rₛ= 0.16, p= 0.001) on the index admission (as defined by EF < 40% or LVWMI > 1.8 on echocardiography) with a stronger relationship demonstrated for NTproBNP (rₛ= 0.35, p= 0.001). Plasma MPO was weakly correlated to PR3-SERPIN A1 and NTproBNP (rₛ= 0.11 and 0.12 respectively, p= 0.002) but not to LV systolic function.

Primary Endpoints: PR3-SERPIN A1 and NTproBNP as predictors of death and heart failure Binary logistic models were developed for prediction of death or heart failure, using the following clinical and demographic variables: age, gender, past history of AMI, hypertension or diabetes, Killip Class > 1, thrombolytic use, log creatinine, NTproBNP, MPO and PR3-SERPIN A1. Independent predictors are reported in Table 2 and include NTproBNP and PR3-SERPIN A1 as significant predictor variables. The Nagelkerke r² was 0.35 suggesting a good fit of the model.

The receiver-operating-characteristic curve for the predictive value of PR3-SERPIN A1 for the primary endpoint yielded an area under the curve (AUC) of 0.65 (95% CI: 0.60-0.70, p<0.001); for NTproBNP the AUC was 0.78 (95% CI: 0.74-0.83, p<0.001). The logistic model combining the 2 markers yielded an AUC of 0.84 (95% CI: 0.80-0.88, p<0.001), which exceeded that of either peptide alone (p<0.003 by method of Hanley and McNeil; Radiology 1983, 148, 839-43).

Cox proportional hazards modelling confirmed these findings, identifying the same independent predictors of death or heart failure apart from gender (Table 3). In both logistic and Cox models, plasma MPO was not an independent predictor of death and heart failure.

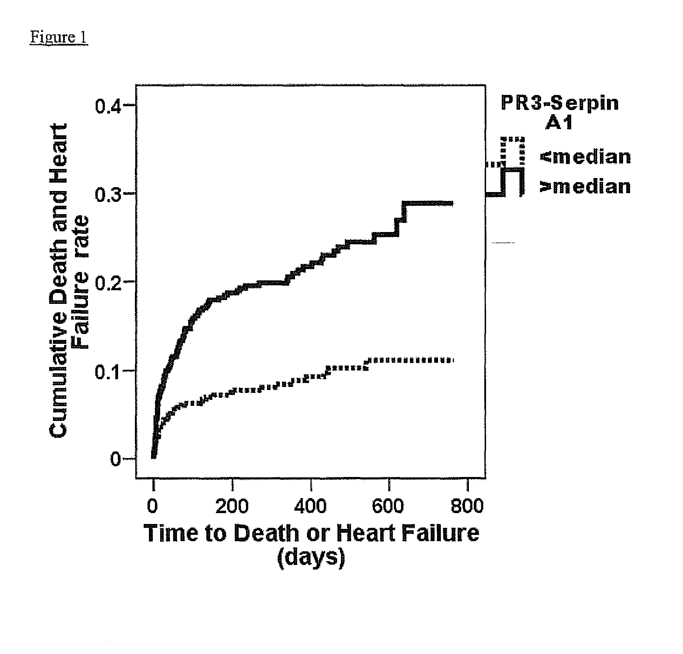

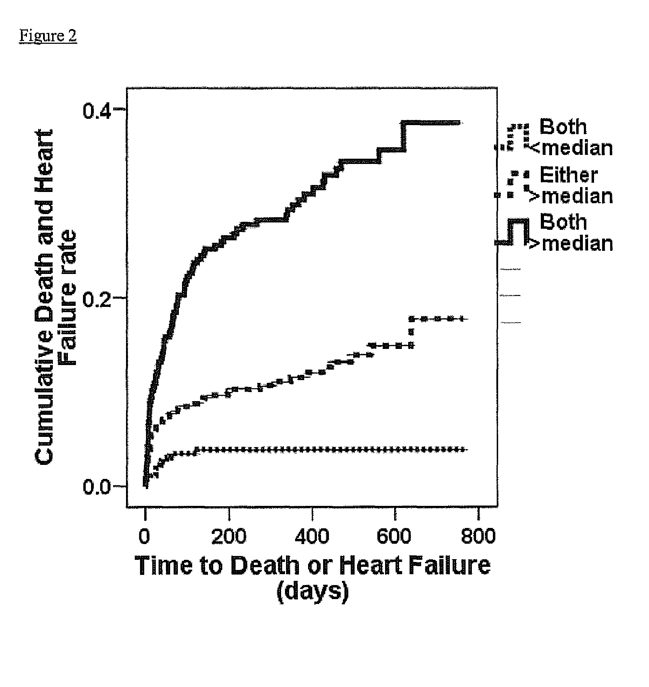

Kaplan-Meier survival analysis demonstrated a significantly better clinical outcome in patients with PR3-SERPIN A1 below the median (504.6 ng/ml) compared with those with PR3-SERPIN A1 above the median (log rank 28.15, p<0.0001, figure 1). The event free survival curves diverge early and continue to diverge even after 2 years. This was also true for NTproBNP (log rank 64.72, p<0.0001). When patients were stratified by plasma NTproBNP (median 1023 pmol/L), PR3-SERPIN A1 gave additional predictive value for death and heart failure, in both the patients in whom NTproBNP level was above the median (log rank for trend 12.54 p<0.0004) and those with NTproBNP level below the median (log rank for trend 3.83 p<0.05). Those patients with both markers elevated above their respective medians had higher event rates than those with either marker elevated above median, and these in turn had higher event rates than those with both markers below their respective medians (p<0.001 for all comparisons, figure 2).

Secondary Endpoints: PR3-SERPIN A1 and NTproBNP as predictors of myocardial infarction.
Compared to survivors with no endpoints, patients who were readmitted with further AMI during follow up had similar NTproBNP (median [range] 1480.7[2.6-10645.9] vs. 806.8[0.3-28886] pmol/L; p=NS), PR3-SERPIN A1 levels (median [range] 482.5[168.9-1753.9] vs. 486.9[29.3-3118.2] ng/ml; p=NS) and MPO levels (23.9 [5.3-189.9] vs 24.6 [0.3-405.2] ng/ml; p=NS).

### Additional markers:

Logistic regression models and Cox proportional hazards models were constructed for prediction of death or of the combined endpoint death or heart failure to assess whether other markers (MR-proANP, CT-proET-1, MR-proADM, Copeptin, NT-proBNP) add prognostic information to that of PR3-SERPIN A1 complex (blood draws of day 4 were assessed). In all analyses all markers assessed (MR-proANP, CT-proET-1, MR-proADM, Copeptin, NTproBNP) added statistically significant prognostic information to that of PR3-SERPIN A1.

**Table 2: Binary Logistic regression model for prediction of death and heart failure.**

| Variable | Odds Ratio | 95% CI | p value |
|---|---|---|---|
| Age | 1.04 | 1.02-1.07 | 0.001 |
| Gender | 0.57 | 0.35-0.93 | 0.024 |
| Past history AMI | 2.28 | 1.43-3.74 | 0.001 |
| Killip Class >1 | 1.66 | 1.05-2.63 | 0.032 |
| Log Creatinine | 18.55 | 3.02-113.9 | 0.001 |
| Log NTproBNP | 2.79 | 1.79-4.37 | 0.001 |
| Log PR3-SERPIN A1 | 6.42 | 2.25-18.3 | 0.001 |

**Table 3: Multivariate Cox proportional hazards regression model of significant predictors of death or heart failure.**

| Variable | Hazard Ratio | 95% CI | p value |
|---|---|---|---|
| Age | 1.04 | 1.03-1.06 | 0.001 |
| Past history AMI | 1.52 | 1.06-2.17 | 0.023 |
| Killip Class >1 | 1.60 | 1.08-2.37 | 0.02 |
| Log Creatinine | 4.75 | 1.39-16.19 | 0.013 |
| Log NTproBNP | 2.51 | 1.70-3.71 | 0.001 |
| Log PR3-SERPIN A1 | 3.80 | 1.78-8.14 | 0.001 |

## Claims

1. A method for risk stratification and/or prognosis and/or therapy control and/or monitoring of a patient having a cardiological disease or condition, comprising:
a) providing a sample from said patient with a cardiological disease or condition,
b) determining the level of a protease from azurophilic granules of polymorphonuclear neutrophils or a complex thereof with an endogenous inhibitor in said sample,
c) attributing the level of said protease from azurophilic granules of polymorphonuclear neutrophils or a complex thereof with an endogenous inhibitor to a prognosis or the degree of severity of the disease or condition or the risk of an adverse outcome for said patient.

2. A method according to claim 1, wherein the level of a protease selected from elastase, cathepsin G, proteinase 3 (Pr3) or a complex thereof with an endogenous inhibitor is determined in step b) and attributed to a prognosis or the degree of severity of the disease or condition or the risk of an adverse outcome for said patient in step c).

3. A method according to claim 1, wherein the level of proteinase 3 or the proteinase 3/Serpin A1 complex is determined in step b) and attributed to a prognosis or the degree of severity of the disease or condition or the risk of an adverse outcome for said patient in step c).

4. A method according to any of the preceding claims, wherein the cardiological disease or condition is selected from the group comprising acute coronary syndrome, including unstable angina (UA), non-ST segment elevation myocardial infarction (NSTEMI) or ST segment elevation myocardial infarction (STEMI).

5. A method according to any of the preceding claims, wherein in step b) the level of said protease or a complex thereof with an endogenous inhibitor in said sample is determined with a diagnostic assay.

6. A method according to any of the preceding claims, wherein the protease from azurophilic granules of polymorphonuclear neutrophils or a complex thereof with an endogenous inhibitor is detected by use of two capture molecules, wherein the two capture molecules may be added to the sample sequentially or simultaneously.

7. A method according to any of the preceding claims, wherein step c) comprises
c1) measuring a signal corresponding to the amount of bound protease or bound complex thereof with an endogenous inhibitor,
c2) comparing the intensity of said signal of step c1) with pre-recorded data, wherein said pre-recorded data correlates levels of the protease or complex thereof with an endogenous inhibitor with a prognosis or the degree of severity of the disease or condition or the risk of an adverse outcome.

8. A method according to claim 7, wherein the level of the protease or complex thereof with an endogenous inhibitor is correlated with a prognosis or the degree of severity of the disease or condition or the risk of an adverse outcome by a method selected from the group comprising correlation with respect to the median of the level of the protease or complex thereof with an endogenous inhibitor in an ensemble of pre-determined samples; correlation with respect to quantiles (e.g. quartiles or percentiles) of the level of the protease or complex thereof with an endogenous inhibitor in an ensemble of pre-determined samples; or correlation with a mathematical model including the level of the protease or complex thereof with an endogenous inhibitor, preferably Cox Regression.

9. A method according to any of the preceding claims, wherein said sample is a plasma sample, a serum sample, a blood sample or fractions thereof, a lymphatic fluid sample, a urine sample or an extract of any of the aforementioned samples.

10. A method according to any of the preceding claims, wherein additionally the level of one or more further markers or clinical parameters, which are associated with or useful for risk stratification and/or prognosis and/or therapy control and/or monitoring of a patient having a cardiological disease or condition is determined.

11. A method according to claim 10, wherein the clinical parameter is a parameter selected from the group comprising age, gender, prior history of diseases, in particular myocardial infarction, Angina Pectoris, hypertension, Diabetes mellitus, hypercholesterolaemia, ST-elevation AMI, body mass index, genetic predisposition / family history, ethnic background, habits which affect said propensity, such as smoking, alcohol consumption, diet, or parameters upon hospitalization, such as Killip Class > 1, or thrombolytic medication upon hospitalization.

12. A method according to claim 10 or 11, wherein the further markers are selected from the group comprising, proBNP or fragments thereof of at least 12 amino acids including BNP and NT-proBNP, proANP or fragments thereof of at least 12 amino acids including NT-proANP and MR-proANP, proAdrenomedullin or fragments thereof of at least 12 amino acids including Adrenomedullin, PAMP and MR-proADM, proEndothelin or fragments thereof of at least 12 amino acids including Endothelin-1, big-Endothelin-1, CT-proET-1 and NT-proET-1, proVasopressin or fragments thereof of at least 8 amino acids including Vasopressin, Copeptin and Neurophysin 2, myeloperoxidase, Troponin, FABP, C-reactive protein, procalcitonin, interleukin-6, ST-2, GDF-15, ischemia modified albumin, or fragments thereof.

13. A method according to claim 12, wherein the further marker is NT-proBNP or a fragment thereof of at least 12 amino acids.

14. Use of a kit comprising at least one capture molecule directed against a protease from azurophilic granules of polymorphonuclear neutrophils and/or a complex thereof with an endogenous inhibitor for detecting and/or determining the level of said protease from azurophilic granules of polymorphonuclear neutrophils and/or a complex thereof with an endogenous inhibitor in said sample for risk stratification and/or therapy control and/or monitoring and or prognosis of a patient having a cardiological disease or condition.

15. The use of a kit according to claim 14, wherein the prognosis or the degree of severity or the disease or condition or the risk of an adverse outcome is determined.

16. The use of a kit according to claim 15 or a method according to any of claims 1 to 11, wherein said adverse outcome is death or heart failure.

17. The use of a protease from azurophilic granules of polymorphonuclear neutrophils or a complex thereof with an endogenous inhibitor as a biomarker for a cardiological disease or condition.
